# EUROPEAN PATENT APPLICATION

(11) **EP 4 847 025 A1**
(43) Date of publication of application: **02.12.2026**
(21) Application number: 25744601.3
(22) Date of filing: 22.01.2025
(51) Int. Cl.: A61K 31/55, A61K 31/517, A61K 31/506, A61K 31/505, A61K 31/416, A61P 35/00

(54) **JOINT USE OF FAK INHIBITOR AND KRAS G12C INHIBITOR**

(30) Priority: 22.01.2024 CN 202410090948; 13.03.2024 CN 202410288108
(71) Applicant: Inxmed (Nanjing) Co., Ltd., Nanjing, Jiangsu 210000 (CN)
(72) Inventor: ZHANG, Baoyuan, Nanjing, Jiangsu 210000 (CN); GAO, Jiaming, Nanjing, Jiangsu 210000 (CN); LIU, Xuebin, Nanjing, Jiangsu 210000 (CN); WANG, Zaiqi, Nanjing, Jiangsu 210000 (CN)
(74) Representative: D Young & Co LLP
(86) International application number: PCT/CN2025/073859
(87) International publication number: WO 2025/157162

(57) **Abstract**

The present invention relates to the use of a FAK inhibitor in preparing a drug to be jointly used with a KRAS G12C inhibitor to treat a tumor.

## Description

### TECHNICAL FIELD

The present disclosure belongs to the field of pharmaceutical chemistry. Specifically, the present disclosure relates to the use of a FAK inhibitor in the manufacture of a medicament for use in combination with a KRAS G12C inhibitor for the treatment of a tumor.

### BACKGROUND

KRAS is the oncogene with the highest mutation frequency during cancer development, and the mutation mainly appears in cancer types such as lung cancer, pancreatic cancer, and colorectal cancer. KRAS was found in the early 1980s. It was not until 2013 that a binding pocket targeting the KRAS G12C mutation was first identified, which keeps KRAS in an inactive state, thereby inhibiting its activity and producing an anti-tumor effect. Based on this principle, various KRAS G12C inhibitors have been developed, among which Sotorasib (also known as AMG510) is already on the market primarily for the treatment of non-small cell lung cancer. However, the aforementioned KRAS G12C inhibitors are first-generation targeted inhibitors, which have a potential problem of limited therapeutic effect. Therefore, there is still a need for a new method for treating cancer, particularly tumors with KRAS mutations.

### SUMMARY

The applicant found that the important mechanism of KRAS G12C inhibitor resistance is the feedback upregulation of the FAK-YAP signaling pathway, thereby evading the apoptotic response induced by targeted therapy. The combined use of AMG510 and FAK inhibitors can generate strong synergistic anti-tumor response. The applicant found the synergistic effect of the second generation KRAS G12C inhibitor and the FAK inhibitor through exploration. This combination regimen will further enhance the synergistic response of the first-generation AMG510 and the FAK inhibitor, thereby benefiting cancer patients, particularly those with the KRAS G12C mutation.

In one aspect, the present disclosure provides the use of a FAK inhibitor in the manufacture of a medicament for use in combination with a KRAS G12C inhibitor for the treatment of a tumor, wherein the FAK inhibitor is Defactinib, AMP945, APG-2449, Conteltinib, a deuterated compound of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

In another aspect, the present disclosure provides the use of a FAK inhibitor and a KRAS G12C inhibitor in the manufacture of a medicament for the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

In another aspect, the present disclosure provides a FAK inhibitor and a KRAS G12C inhibitor for use in the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

In another aspect, the present disclosure provides a method of treating a tumor comprising administering to a subject an FAK inhibitor and a KRAS G12C inhibitor, wherein the FAK inhibitor is Defactinib, AMP945, APG-2449, Conteltinib, deuterated compounds of Defactinib, deuterated compounds of AMP945, deuterated compounds of APG-2449, or a pharmaceutically acceptable salt thereof, and the KRAS G12C inhibitor is RMC-4998, Divarasib, LY3537982, Opnurasib, deuterated compounds of Conteltinib, or a pharmaceutically acceptable salt thereof, particularly a therapeutically effective amount of the FAK inhibitor and a therapeutically effective amount of the KRAS G12C inhibitor.

In another aspect, the present disclosure provides a pharmaceutical combination product comprising:
(a) a FAK inhibitor, and
(b) a KRAS G12C inhibitor,
wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

Alternatively, the FAK inhibitor is Defactinib, AMP945, APG-2449, Conteltinib, deuterated compound 1 of Defactinib (CAS No. 2384121-03-1), deuterated compound 2 of Defactinib (CAS No. 2384120-99-2), or a pharmaceutically acceptable salt thereof.

Alternatively, the FAK inhibitor is Defactinib, AMP945, APG-2449, or deuterated compounds of Conteltinib.

Alternatively, the FAK inhibitor is administered simultaneously, separately or sequentially with the KRAS G12C inhibitor.

Alternatively, the FAK inhibitor is administered at a dose of about 25 mg/day to 1000 mg/day, and further, the FAK inhibitor is administered at a dose of about 25 mg/day to 500 mg/day.

Alternatively, the KRAS G12C inhibitor is administered at a dose of about 100 mg/day to 2000 mg/day.

Alternatively, the tumor is a tumor with a KRAS mutation, and further is a tumor with a KRAS G12C mutation.

Alternatively, the tumor is lung adenocarcinoma, non-small cell lung cancer, colorectal cancer (CRC) (including colon cancer and rectal cancer), endometrial cancer, bladder urothelial carcinoma, invasive lobular carcinoma of the breast, cervical squamous cell carcinoma, melanoma of the skin, endocervical adenocarcinoma, hepatocellular carcinoma, pancreatic cancer, biphasic pleural mesothelioma, clear cell carcinoma of kidney, gastric adenocarcinoma, tubular gastric adenocarcinoma, uterine carcinosarcoma, or malignant mixed Mullerian tumor of the uterus.

Alternatively, the tumor is non-small cell lung cancer.

### BRIEF DESCRIPTION OF DRAWINGS

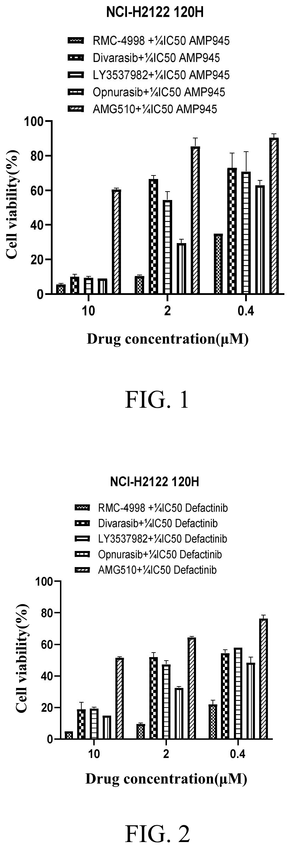

FIG. 1 depicts the effect of different concentrations of KRAS G12C inhibitor in combination with AMP945 on the proliferation of NCI-H2122 cells. Three different drug concentrations were selected for KRAS G12C inhibitor: 10 µM, 2 µM, and 0.4 µM. A fixed concentration of ¼IC50 was selected for AMP945.
FIG. 2 depicts the effect of different concentrations of KRAS G12C inhibitor in combination with Defactinib on the proliferation of NCI-H2122 cells. Three different drug concentrations were selected for KRAS G12C inhibitor: 10 µM, 2 µM, and 0.4 µM. A fixed concentration of ¼IC50 was selected for Defactinib.

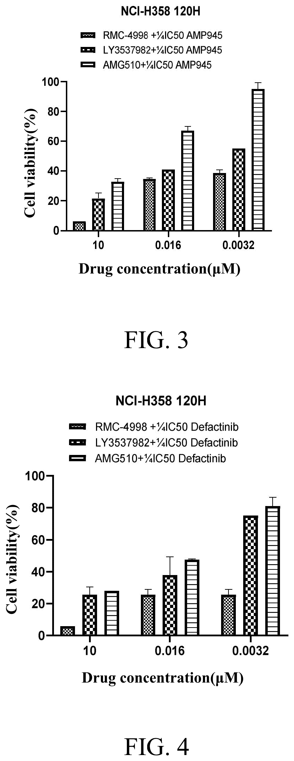

FIG. 3 depicts the effect of different concentrations of KRAS G12C inhibitor in combination with AMP945 on the proliferation of NCI-H358 cells. Three different drug concentrations were selected for KRAS G12C inhibitor: 10 µM, 0.016 µM, and 0.0032 µM. A fixed concentration of ¼IC50 was selected for AMP945.
FIG. 4 depicts the effect of different concentrations of KRAS G12C inhibitor in combination with Defactinib on the proliferation of NCI-H358 cells. Three different drug concentrations were selected for KRAS G12C inhibitor: 10 µM, 0.016 µM, and 0.0032 µM. A fixed concentration of ¼IC50 was selected for Defactinib.

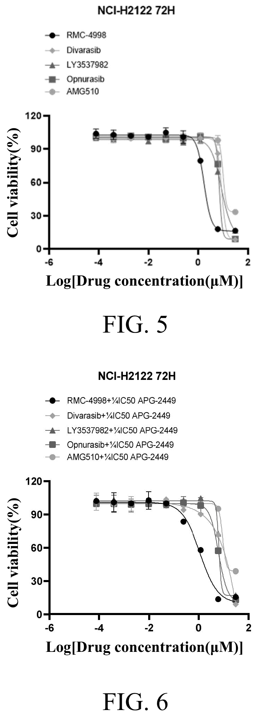

FIG. 5 depicts the effect of different concentrations of KRAS G12C inhibitor on the proliferation of NCI-H2122 cells.
FIG. 6 depicts the effect of different concentrations of KRAS G12C inhibitor in combination with APG-2449 on the proliferation of NCI-H2122 cells. The concentration of APG-2449 was ¼IC50, and the IC50 of APG-2449 was 3.5 µM.

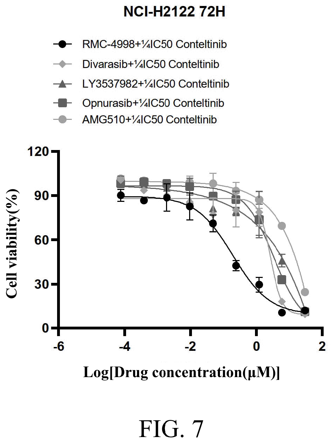

FIG. 7 depicts the effect of different concentrations of the KRAS G12C inhibitor in combination with Conteltinib on the proliferation of NCI-H2122 cells. The concentration of Conteltinib was ¼IC50, and the IC50 of Conteltinib was 7 µM.

### DETAILED DESCRIPTION

To make the objects, technical solutions and advantages of the embodiments of the present disclosure clearer, the technical solutions of the embodiments of the present disclosure will be clearly and fully described below with reference to the accompanying drawings of the embodiments of the present disclosure. It is obvious that the described embodiments are just some, but not all of the embodiments of the present disclosure. Based on the described embodiments of the present disclosure, all other embodiments obtained by those of ordinary skill in the art without creative effort will fall within the scope of protection of the present disclosure.

The present disclosure can be implemented in other specific forms without departing from the spirit of the present disclosure. It should be understood that any and all embodiments of the present disclosure can be combined with the technical feature(s) of any other embodiment(s) to obtain alternative embodiments on the premise of no conflict. The disclosure encompasses such combination to obtain alternative embodiments.

All the publications and patents mentioned in the present disclosure are hereby incorporated by reference in their entirety. If the use or terminology used in any publication or patent incorporated by reference conflicts with the use or terminology used in the present disclosure, the use and terminology of this disclosure shall prevail.

The section headings used herein are used for organizational purposes only and are not to be construed as limiting the subject matter described.

Unless otherwise specified, all technical and scientific terms used herein have the same meaning as those commonly used in the art to which the claimed subject matter belongs. If there are multiple definitions for a term, the definition herein shall prevail.

Unless in the working examples or otherwise indicated, all numbers stating quantitative properties such as dosage in the specification and claims should be understood as modified by the term "about" in all instances. "About" and "approximately" generally mean the extent of acceptable error of the amount measured taking into account the nature or accuracy of the measurement. Exemplary degrees of error are within 20 percent (%) of a given value or range of values, typically within 10%, and more typically within 5%. It should also be understood that any numerical range enumerated in the present application is intended to include all sub-ranges within that range and any combination of the endpoints of that range or sub-ranges. For example, the KRAS G12C inhibitor is administered at about 100 mg/day to about 2000 mg/day, for example, about 100 mg/day to about 1500 mg/day, about 100 mg/day to about 1000 mg/day, about 100 mg/day to about 800 mg/day, about 100 mg/day to about 600 mg/day, about 100 mg/day to about 400 mg/day, about 100 mg/day to about 200 mg/day, about 200 mg/day to about 2000 mg/day, about 200 mg/day to about 1500 mg/day, about 200 mg/day to about 1000 mg/day, about 200 mg/day to about 800 mg/day, about 200 mg/day to about 600 mg/day, about 200 mg/day to about 400 mg/day, about 400 mg/day to about 2000 mg/day, about 400 mg/day to about 1500 mg/day, about 400 mg/day to about 1000 mg/day, about 400 mg/day to about 800 mg/day, about 400 mg/day to about 600 mg/day, about 600 mg/day to about 2000 mg/day, about 600 mg/day to about 1500 mg/day, about 600 mg/day to about 1000 mg/day, about 600 mg/day to about 800 mg/day, about 800 mg/day to about 2000 mg/day, about 800 mg/day to about 1500 mg/day, about 800 mg/day to about 1000 mg/day, about 900 mg/day to about 2000 mg/day, about 900 mg/day to about 1500 mg/day, about 900 mg/day to about 1000 mg/day. In some embodiments, the KRAS G12C inhibitor is administered at about 100 mg/day. In some embodiments, the KRAS G12C inhibitor is administered at about 200 mg/day. In some embodiments, the KRAS G12C inhibitor is administered at about 300 mg/day. In some embodiments, the KRAS G12C inhibitor is administered at about 400 mg/day. In some embodiments, the KRAS G12C inhibitor is administered at about 500 mg/day. In some embodiments, the KRAS G12C inhibitor is administered at about 600 mg/day. In some embodiments, the KRAS G12C inhibitor is administered at about 700 mg/day. In some embodiments, the KRAS G12C inhibitor is administered at about 800 mg/day. In some embodiments, the KRAS G12C inhibitor is administered at about 900 mg/day. In some embodiments, the KRAS G12C inhibitor is administered at about 1000 mg/day.

The terms "comprise/comprising," "include/including," or "contain/containing," or similar terms are intended to specify that the elements stated before these terms encompass the elements and equivalents thereof listed after these terms, but do not preclude the elements not recited. The term "comprise," "include (contain)" used herein can be in open, semi-closed, and closed forms. In other words, the term also comprises "consisting essentially of" or "consisting of."

The experimental materials and reagents used in the following examples can be commercially available, unless otherwise specified.

The present disclosure relates to use of a FAK inhibitor in the manufacture of a medicament for use in combination with a KRAS G12C inhibitor for the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

The present disclosure also relates to the use of a FAK inhibitor and a KRAS G12C inhibitor in the manufacture of a medicament for the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

The present disclosure also relates to a FAK inhibitor and a KRAS G12C inhibitor for use in the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

The present disclosure also relates to a method of treating a tumor comprising administering to a subject an FAK inhibitor and a KRAS G12C inhibitor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof, particularly a therapeutically effective amount of the FAK inhibitor and a therapeutically effective amount of the KRAS G12C inhibitor.

The present disclosure also relates to a pharmaceutical combination product comprising: (a) a FAK inhibitor, and (b) a KRAS G12C inhibitor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

In some embodiments, the FAK inhibitor is Defactinib, AMP945, APG-2449, Conteltinib, deuterated compound 1 of Defactinib (CAS No. 2384121-03-1), deuterated compound 2 of Defactinib (CAS No. 2384120-99-2), or a pharmaceutically acceptable salt thereof. In some embodiments, the FAK inhibitor is Defactinib, AMP945, APG-2449, or deuterated compounds of Conteltinib.

The FAK inhibitor, an effective inhibitor of FAK protein tyrosine kinase, can be suitable for therapeutic use in mammals, particularly in humans, as an anti-proliferative agent (e.g., an anticancer agent), an anti-tumor agent (e.g., effective against solid tumors), and an antiangiogenic agent (e.g., stopping or preventing vascular proliferation). The compounds described herein, such as FAK inhibitors, are useful in the prevention and treatment of diseases or conditions described herein (e.g., cancer). The compounds described herein, such as FAK inhibitors, are useful in the prevention and treatment of non-hematologic malignancies, various human hyperproliferative diseases, such as the following malignant and benign tumors: liver cancer, kidney caner, bladder cancer, breast cancer, stomach cancer, ovary cancer, colorectal cancer, prostate cancer, pancreatic cancer, lung cancer, vulvar cancer, thyroid cancer, hepatocellular carcinoma, sarcoma, glioblastoma, head and neck cancer, and other proliferative diseases such as benign hyperplasia of the skin (e.g., psoriasis) and benign hyperplasia of the prostate (e.g., BPH), as well as the prevention and treatment of diseases such as mesothelioma.

Defactinib, which has the following structure, is also known as VS-6063 (e.g., VS-6063 free base) or PF-04554878. VS-6063 and related compounds are also disclosed, for example, in U.S. Patent No. 7,928,109, the contents of which are incorporated herein by reference. In some embodiments, VS-6063 may form a pharmaceutically acceptable salt (e.g., hydrochloride salt),

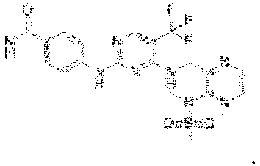

Defactinib has a variety of deuterated derivatives, such as deuterated compound 1 of Defactinib (CAS No. 2384121-03-1) and deuterated compound 2 of Defactinib (CAS No. 2384120-99-2).

AMP945 has the following structure, whose synthesis and characterization are described in WO2012110773. In some embodiments, AMP945 may form a pharmaceutically acceptable salt (e.g., tartrate),

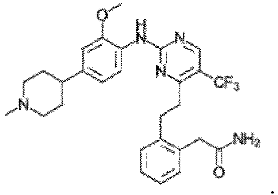

The CAS No. of APG-2449 is 2196186-84-0.

Conteltinib, also known as CT-707, has the CAS No. 1384860-29-0.

Exemplary KRAS G12C inhibitors are as follows:
RMC-4998, with the CAS No. of 2642037-07-6;
Divarasib, with the CAS No. of 2417987-45-0;
LY3537982, with the CAS No. of 2414198-64-2;
Opnurasib, with the CAS No. of 2653994-08-0;
AMG510, with the CAS No. of 2296729-00-3.

As used herein, "combination use", "combination therapy", and "drug combination" refers to the use of a drug in combination with one or more other drugs to treat a disease, including the combination of a drug with one or more other drugs and the combination of a drug with instructions indicating that the drug can be used in combination with one or more other drugs.

As used herein, "pharmaceutical combination" or "pharmaceutical combination product" can refer to a fixed combination in the form of one dosage unit (for example, all active pharmaceutical ingredients are present in one dosage form) or a product as a kit-of-parts for combined administration, or it may refer to a combination of one drug and instructions indicating that the drug can be used in combination with one or more other drugs.

"Synchronously, alternately, or sequentially" in the present application refers to the simultaneous or sequential administration at certain time intervals of more than two drugs within one administration cycle (e.g., within 4 weeks, within 3 weeks, within 2 weeks, within 1 week, or within 24 hours). The modes of drug administration (e.g., oral, intravenous, intramuscular, or subcutaneous administration, etc.) may be the same or different, and the dosing frequency/cycle of more than two drugs may be the same or different. When the treatment method, product, or use of the present disclosure involves two drugs, the two drugs may be administered simultaneously or separately at certain time intervals.

As used herein, the term "treatment" refers to achieving a desired pharmacological and/or physiological effect. The effect may be therapeutic, including in part or substantially obtaining one or more of the following: partially or completely reducing the degree of disease, condition or syndrome; improving clinical symptoms or indicators associated with the disease; or delaying, inhibiting or reducing the likelihood of disease, condition or syndrome progression.

As used herein, the term "tumor" refers to an abnormal lesion formed by the loss of normal regulation of local tissue cells' growth at the genetic level under the influence of various tumorigenic factors, resulting in abnormal clonal proliferation. For example, the tumor includes, but are not limited to: lung adenocarcinoma, non-small cell lung cancer, colorectal cancer (CRC) (including colon cancer and rectal cancer), endometrial cancer, bladder urothelial carcinoma, invasive lobular carcinoma of the breast, cervical squamous cell carcinoma, melanoma of the skin, endocervical adenocarcinoma, hepatocellular carcinoma, pancreatic cancer, biphasic pleural mesothelioma, clear cell carcinoma of kidney, gastric adenocarcinoma, tubular gastric adenocarcinoma, uterine carcinosarcoma, or malignant mixed Mullerian tumor of the uterus.

As used herein, the term "pharmaceutically acceptable" refers to non-toxic, biologically tolerable, and suitable for administration to subjects.

As used herein, the term "pharmaceutically acceptable salts" refer to non-toxic, biologically tolerable salts suitable for administration to a subject. For example, a pharmaceutically acceptable salt of a compound of formula (I) refers to a non-toxic, biologically tolerable acid addition salts suitable for administration to a subject, including but not limited to: acid addition salts formed by the compound with inorganic acids, such as hydrochloride, hydrobromide, carbonate, bicarbonate, phosphate, sulfate, sulfite, nitrate, etc.; and acid addition salts formed by the compound with organic acids, such as formate, acetate, malate, maleate, fumarate, tartrate, succinate, citrate, lactate, methanesulfonate, p-toluenesulfonate, 2-hydroxyethanesulfonate, benzoate, salicylate, stearate, and salts formed with alkanedicarboxylic acids of the formula HOOC-(CH₂)ₙ-COOH (wherein n is 0-4), etc. Pharmaceutically acceptable salts can be obtained by conventional methods well known in the art, for example by reacting a sufficiently basic compound, such as an amine, with a suitable acid that provides a physiologically acceptable anion. In some embodiments, the pharmaceutically acceptable salt is a tartrate.

As used herein, the term "patient" or "subject" refers to both mammals and non-mammals. A mammals refers to any member of the mammal family, including but not limited to human beings; non-human primates such as chimpanzee and other apes and monkey species; farm animals such as cattle, horses, sheep, goats, and pigs; livestock such as rabbits, dogs, and cats; laboratory animals, including rodent such as rats, mice, and guinea pigs; and the like. Examples of non-mammals include, but are not limited to, birds. The term "subject" does not limit to a particular age or sex. In some embodiments, the subject is a human.

As used herein, the term "effective amount" refers to a FAK inhibitor, or a KRAS G12C inhibitor, in an amount sufficient to reduce or improve the severity, duration, or onset of a disease or condition, delay or prevent the progression of a disease or condition, induce the remission of a disease or condition or delay the recurrence or progression of symptoms, or enhance or improve the therapeutic effect of another therapy. The exact amount of FAK inhibitor or KRAS G12C inhibitor administered to a subject will depend on various factors, such as the given drug or compound, the drug preparation, the route of administration, the type of disease, the condition, the identity of the subject or host being treated, etc., but can nevertheless be routinely determined by one skilled in the art. For example, the determination of an effective amount will also depend on the extent, severity, and type of cell proliferation. A skilled artisan will be able to determine an appropriate dosage based on these and other factors. When co-administered with other therapeutic agents, for example, when co-administered with an anticancer agent, the "effective amount" of any other therapeutic agent will depend on the type of drug used. A suitable amount is known for an approved therapeutic agent and may be adjusted by the skilled artisan depending on the condition of the subject, the type of condition being treated, and the amount of the compound or a pharmaceutically acceptable salt thereof. In the absence of a clearly specified quantity, it should be assumed that there is some amount.

The effective amount of the FAK inhibitor may be, for example, 10 mg - 2000 mg/day. In some embodiments, the effective amount of the FAK inhibitor may be, for example, 25 mg - 1000 mg/day; in some embodiments, the effective amount of the FAK inhibitor may be, for example, 25 mg - 500 mg/day.

The effective amount of the KRAS G12C inhibitor may be, for example, 10 mg - 2000 mg/day. In some embodiments, the effective amount of the KRAS G12C inhibitor may be, for example, 25 mg - 1000 mg/day; in some embodiments, the effective amount of the KRAS G12C inhibitor may be, for example, 100 mg - 2000 mg/day.

The administration dosage and administration regimen of the FAK inhibitor, or the KRAS G12C inhibitor required for the treatment can vary not only with the particular salt selected, but also with the route of administration, the nature of the disease being treated, the age and physical condition of the patient and whether there are concurrent chronic diseases of other organs, and may ultimately be determined by the attending physician or clinician at their own discretion.

### EXAMPLE

The following examples are provided to further describe the present disclosure. It should also be understood that these examples are only for the purpose of illustrating the present disclosure, rather than limiting the scope thereof.

The experimental methods without specific conditions in the following examples can be carried out according to the conventional conditions of this type of reaction or the conditions suggested by the manufacturer.

The experimental materials and reagents used in the following examples are commercially available unless otherwise specified.

### Example 1: A study on the inhibitory activity of KRAS G12C inhibitor in combination with three different FAK inhibitors on cell proliferation in vitro.

NCI-H2122/NCI-H358 (non-small cell lung cancer cell line) cells were resuscitated and maintained for passage by Inxmed (Nanjing) Co., Ltd. Cells were cultured in vitro in an adherent monolayer under the following culture conditions: RPMI1640 medium supplemented with 10% fetal bovine serum, at 37 °C and 5% CO₂, and the cells were trypsinized and passaged two to three times per week. When the cells were in the exponential growth phase, and the adherent confluence reached 80%-90%, they were harvested and plated.

The NCI-H2122/NCI-H358 cells were trypsinized, and then the cells were collected and counted. Based on the count results, the cells were diluted to 30,000 cells per milliliter using the corresponding culture medium. The cells were then plated in a 96-well cell flat-bottomed culture plate, with 0.1 ml of cell suspension (i.e., 3,000 cells) added to each well. After the cell plating was completed, the culture plates were placed in a 37°C, 5% CO₂ incubator for further culture.

24 hours after cell plating, the test compounds were added to different wells of the cell plate. The grouping is shown in Tables 1 and 2, and the information on the test compounds is shown in Table 3. After the drugs were added, they were gently mixed, and then the cells were incubated in a 37 °C, 5% CO₂ incubator.

**Table 1: Grouping scheme for NCI-H2122 cell proliferation inhibition assay**

| **Number** | **Test Drug** |
|---|---|
| 1 | RMC-4998 +¼IC50 AMP945 |
| 2 | Divarasib+¼IC50 AMP945 |
| 3 | LY3537982+¼IC50 AMP945 |
| 4 | Opnurasib+¼IC50 AMP945 |
| 5 | AMG510+¼IC50 AMP945 |
| 6 | RMC-4998 +¼IC50 Defactinib |
| 7 | Divarasib+¼IC50 Defactinib |
| 8 | LY3537982+¼IC50 Defactinib |
| 9 | Opnurasib+¼IC50 Defactinib |
| 10 | AMG510+¼IC50 Defactinib |

The IC50s of AMP945 and Defactinib previously detected on CT26 cells were 6.2 µM and 6.9 µM, respectively. Therefore, the ¼IC50 for AMP945 in this experiment was 1.55 µM, and the ¼IC50 for Defactinib was 1.72 µM.

**Table 2: Grouping scheme for NCI-H358 cell proliferation inhibition assay**

| **Number** | **Test Drug** |
|---|---|
| 1 | RMC-4998 +¼IC50 AMP945 |
| 2 | LY3537982+¼IC50 AMP945 |
| 3 | AMG510+¼IC50 AMP945 |
| 4 | RMC-4998 +¼IC50 Defactinib |
| 5 | LY3537982+¼IC50 Defactinib |
| 6 | AMG510+¼IC50 Defactinib |

**Table 3:**

| Name | RMC-4998 | Divarasib | LY3537982 | Opnurasib | AMG510 | AMP945 | Defactinib |
|---|---|---|---|---|---|---|---|
| Code name | RMC-4998 | Divarasib | LY3537982 | Opnurasib | AMG510 | AMP945 | Defactinib |
| Supplier | MCE | MCE | MCE | MCE | Selleck | MCE | Shanghai Chaolan Chemical Co., LTD |
| Batch number | Cat: HY-156671 | Cat: HY-145928 | Cat: HY-132980 | Cat: HY-139612 | Lot: S883001 | Cat: HY-145652 | Lot: 202009 |
| | Lot: 317630 | Lot: 265847 | Lot: 263272 | Lot: 263272 | | Lot:143253 | |
| Vehicle | DMSO | DMSO | DMSO | DMSO | DMSO | DMSO | DMSO |
| Storage conditions | -20°C | -20°C | -20°C | -20°C | -20°C | -20°C | -20°C |

After 120 hours of drug treatment, 10 µL of CCK-8 assay reagent was added to each well of the cell plate using a multi-channel pipette. The cell plate was then placed in a 5% CO2 incubator at 37 °C for further incubation for 4 hours. Finally, the absorbance of each well at 450 nm was measured using a microplate reader.

After the experiment was completed, GraphPad Prism 8 software was used to analyze the percent inhibition of cells by the test drug.

The percent inhibition was calculated as follows: 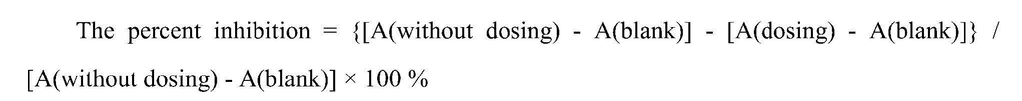 The percent inhibition = {[A(without dosing) - A(blank)] - [A(dosing) - A(blank)]} / [A(without dosing) - A(blank)] × 100 %
A (dosing): Absorbance values of wells containing cells, CCK-8 solution, and drug solution.
A (blank): Absorbance value of wells containing culture medium and CCK-8 solution, but without cells.
A (without dosing): Absorbance value of wells containing cells and CCK-8 solution, but without drug solution.

The results of each group are shown in FIG. 1 to FIG. 4. It can be seen that the combined use of each group showed better cell-killing effects than the combination with AMG510.

### Example 2: A study on the inhibitory activity of KRAS G12C inhibitor in combination with two different FAK inhibitors on cell proliferation in vitro.

Except that some of the test compounds are different, everything else is the same as in Example 1.

The grouping is shown in Table 3.

**Table 3:**

| **Number** | **Test Drug** |
|---|---|
| 1 | RMC-4998 |
| 2 | Divarasib |
| 3 | LY3537982 |
| 4 | Opnurasib |
| 5 | AMG510 |
| 6 | RMC-4998 +¼IC50 APG-2449 |
| 7 | Divarasib+¼IC50 APG-2449 |
| 8 | LY3537982+¼IC50 APG-2449 |
| 9 | Opnurasib+¼IC50 APG-2449 |
| 10 | AMG510+¼IC50 APG-2449 |
| 11 | RMC-4998 +¼IC50 Conteltinib |
| 12 | Divarasib+¼IC50 Conteltinib |
| 13 | LY3537982+¼IC50 Conteltinib |
| 14 | Opnurasib+¼IC50 Conteltinib |
| 15 | AMG510+¼IC50 Conteltinib |

Part of the information of the test compounds is shown in Table 4.

**Table 4:**

| | | |
|---|---|---|
| Name | APG-2449 | Conteltinib |
| Code name | APG-2449 | Conteltinib |

| Supplier | Synthesize according to the method in WO2018044767 | MCE |
|---|---|---|
| Batch number | | Cat: HY-109084, Lot:47089 |
| Vehicle | DMSO | DMSO |
| Storage conditions | 4 °C | -20 °C |

The results for each group are shown in FIG. 5 to FIG. 7. It can be seen that the combined use of each group showed better cell-killing effects than the combination with AMG 510.

Based on the above description, those skilled in the art can readily determine the essential features of the present disclosure, and various changes and modifications can be made to adapt it to various uses and conditions without departing from the spirit and scope of the disclosure. Therefore, other embodiments are also within the scope of the appended claims.

## Claims

1. Use of a FAK inhibitor in the manufacture of a medicament for use in combination with a KRAS G12C inhibitor for the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

2. Use of a FAK inhibitor and a KRAS G12C inhibitor in the manufacture of a medicament for the treatment of a tumor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

3. A FAK inhibitor and a KRAS G12C inhibitor for use in the treatment of a tumor, wherein the FAK inhibitor is Defactinib, AMP945, APG-2449, deuterated compounds of Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

4. A method of treating a tumor comprising administering to a subject an FAK inhibitor and a KRAS G12C inhibitor, wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

5. The use, FAK inhibitor and KRAS G12C inhibitor or method of any one of claims 1-4, wherein the FAK inhibitor is Defactinib, AMP945, APG-2449, Conteltinib, deuterated compound 1 of Defactinib (CAS No. 2384121-03-1), deuterated compound 2 of Defactinib (CAS No. 2384120-99-2), or a pharmaceutically acceptable salt thereof, and further, the FAK inhibitor is Defactinib, AMP945, APG-2449 or Conteltinib.

6. The use, FAK inhibitor and KRAS G12C inhibitor or method of any one of claims 1-5, wherein the FAK inhibitor is administered simultaneously, separately or sequentially with KRAS G12C inhibitor.

7. The use, FAK inhibitor and KRAS G12C inhibitor or method of any one of claims 1-6, wherein the FAK inhibitor is administered at a dose of about 25 mg/day to 1000 mg/day, and further, the FAK inhibitor is administered at a dose of about 25 mg/day to 500 mg/day.

8. The use, FAK inhibitor and KRAS G12C inhibitor or method of claim 7, wherein the KRAS G12C inhibitor is administered at a dose of about 100 mg/day to 2000 mg/day.

9. The use, FAK inhibitor and KRAS G12C inhibitor or method of any one of claims 1-8, wherein the tumor is a tumor with KRAS mutation, and further, the tumor is a tumor with KRAS G12C mutation.

10. The use, FAK inhibitor and KRAS G12C inhibitor or method of any one of claims 1-9, wherein the tumor is lung adenocarcinoma, non-small cell lung cancer, colorectal cancer (CRC) (including colon cancer and rectal cancer), endometrial cancer, bladder urothelial carcinoma, invasive lobular carcinoma of the breast, cervical squamous cell carcinoma, melanoma of the skin, endocervical adenocarcinoma, hepatocellular carcinoma, pancreatic cancer, biphasic pleural mesothelioma, clear cell carcinoma of kidney, gastric adenocarcinoma, tubular gastric adenocarcinoma, uterine carcinosarcoma, or malignant mixed Mullerian tumor of the uterus.

11. The use, FAK inhibitor and KRAS G12C inhibitor or method of claim 10, wherein the tumor is non-small cell lung cancer.

12. A pharmaceutical combination product comprising:
(a) a FAK inhibitor, and
(b) a KRAS G12C inhibitor,
wherein the FAK inhibitor is Defactinib, AMP945, APG-2449, Conteltinib, deuterated compounds of Defactinib, deuterated compounds of AMP945, deuterated compounds of APG-2449, deuterated compounds of Conteltinib, or a pharmaceutically acceptable salt thereof, and the KRAS G12C inhibitor is RMC-4998, Divarasib, LY3537982, Opnurasib, or a pharmaceutically acceptable salt thereof.

13. The pharmaceutical combination product of claim 12, wherein the FAK inhibitor is Defactinib, AMP945, APG-2449, Conteltinib, deuterated compound 1 of Defactinib (CAS No. 2384121-03-1), deuterated compound 2 of Defactinib (CAS No. 2384120-99-2), or a pharmaceutically acceptable salt thereof, and further, the FAK inhibitor is Defactinib, AMP945, APG-2449, or Conteltinib.
